# EUROPÄISCHE PATENTSCHRIFT

(11) **EP 3 338 810 B1**
(45) Veröffentlichungstag und Bekanntmachung des Hinweises auf die Patenterteilung: **23.10.2019**
(21) Anmeldenummer: 17204651.8
(22) Anmeldetag: 30.11.2017
(51) Int. Cl.: A61L 2/24, F26B 5/04, A61L 2/07

(54) **VERFAHREN UND VORRICHTUNG ZUR TROCKNUNG IM DAMPFEXPLOSIONSVERFAHREN**
METHOD AND DEVICE FOR DRYING IN STEAM EXPLOSION METHOD
PROCÉDÉ ET DISPOSITIF DE SÉCHAGE DANS LE PROCÉDÉ D'EXPLOSION DE VAPEUR

(30) Priorität: 05.12.2016 DE 102016123490
(43) Veröffentlichungstag der Anmeldung: 27.06.2018
(73) Patentinhaber: Innovations-Transfer Uphoff GmbH & Co. KG, 35039 Marburg an der Lahn (DE)
(72) Erfinder: Jungkind, Kurt, 63674 Altenstadt (DE)
(74) Vertreter: von Bülow & Tamada

(56) Entgegenhaltungen:
- EP-A1- 0 301 117
- WO-A2-2006/063569
- DE-A1- 19 638 750
- DE-C- 825 829
- DE-C- 887 177
- DE-U1- 29 710 519
- GB-A- 2 374 917

## Beschreibung

Die Erfindung bezieht sich auf ein Verfahren zur schnellen Trocknung insbesondere thermolabiler Oberflächen gemäß dem Oberbegriff des Patentanspruchs 1 sowie auf eine Vorrichtung zur Trocknung durch Erzielung einer explosionsartigen Verdampfung von Flüssigkeiten bzw. Flüssigkeitstropfen gemäß Patentanspruch 3. EP0301117 offenbart eine Einrichtung zur Gefriertrocknung mit einer evakuierbaren Kammer und ein Evakuierungssystem; diese Einrichtung ist mit einer zusätzlichen Kondensationsfläche ausgerüstet, die über ein Ventil an einen Vorratsbehälter mit einem niedrigsiedenden Kältemittel angeschlossen ist; fällt infolge eines Störfalles die Pumpleistung des Evakuierungssystems aus, wird das Ventil geöffnet, so dass die zusätzliche Kondensationsfläche die Pumpwirkung übernimmt.

DE825829 offenbart eine Schleusenventilsteuerung für Vakuumapparate mit einem zum Beschicken und Entleeren verwendbaren Ventilpaar je Schleuse.

Zur Aufbereitung medizinischer Instrumente, labortechnischer Geräte sowie industrieller Zwischen- und Endprodukte kommen Reinigungs-/Trocknungs- sowie Reinigungs- und Desinfektionsgeräte (RDG) zum Einsatz, wie diese seit mehreren Jahrzehnten bekannt sind (vgl. DE 3018050 A1, DE 102007003894 A1, DE 19627762 B4) und in ihrer Grundstruktur den klassischen Haushaltsspülmaschinen entsprechen.

Dabei werden beispielsweise im Human- und Veterinärmedizinischen Bereich (Krankenhäuser, ambulante OP-Zentren, Arztpraxen etc.) unter anderem chirurgische Instrumente, Operationsbestecke, Endoskope sowie Schläuche und Ventilsysteme von Beatmungs- und Narkosegeräten behandelt.

Haushalts-Spülmaschinen, ebenso wie Reinigungs- und Desinfektionsautomaten, verfügen dabei üblicherweise über Spülgutträger, die z.B. als fahrbare Korbwagen mit dem zu reinigenden und zu trocknenden Material (Spülgut) bestückt sind und durch eine Tür in den Spülraum eingeschoben werden.

RDG bearbeiten programmgesteuert mehrere aufeinanderfolgende Arbeitsschritte, wie z.B. Vorspülen, Reinigen, Neutralisieren, Nachspülen und thermische Desinfektion unter Anwendung erhitzten Wassers. Bei einigen dieser Prozessschritte, wie Reinigen oder Neutralisieren, werden Chemikalien zugesetzt, die als Reinigungsflotte mit Druck auf die zu reinigenden Gegenstände aufgebracht werden.

In den weiteren Prozessschritten, wie z.B. Nachspülen, werden verbliebene Reste und Chemikalien aus der Reinigung bzw. Neutralisation mit enthärtetem oder voll entsalztem Spülwasser (VE-Wasser) möglichst vollständig entfernt.

Während des Reinigungs- und Desinfektionsprozesses gelangt die notwendige Spül- und/oder Reinigungs- und/oder Desinfektionsflüssigkeit und/oder Trocknungsluft über Kupplungsvorrichtungen in den Spülgutträger, wie z.B. einen Korbwagen, und wird mittels Düsen, Dreharmen oder drehbaren vergleichbaren Sprüheinrichtungen mit Druck auf das zu reinigende Spülgut geführt. Nach Abschluss des Aufbereitungsprozesses einschließlich erfolgter Trocknung wird der Korbwagen dann wieder durch die Tür (Frontlader) oder durch eine gegenüberliegende zweite Tür (Durchlader) aus dem Spül- bzw. Trocknungsraum herausgezogen.

Aus Gründen des Arbeitsschutzes kann, insbesondere bei RDG, für die medizinische und labortechnische Aufbereitung nach der Reinigung eine Desinfektion mit Heißwasser (z.B. 90-95°C) oder Dampf (z.B. 105°C) erforderlich werden.

Die abschließende Trocknung ist z.B. für RDG in der DIN EN ISO 15883 als allgemein anerkannte Regel der Technik implementiert. Die Anforderungen an die Trocknungsqualität im Sinne der Entfernung von Spülwasserresten sind beispielsweise über das deutsche Medizinproduktegesetz, der zugehörigen Betreiberverordnung, der EWG-Richtlinie 93/42, der DIN EN 285 sowie insbesondere DIN EN ISO 15883 geregelt und detailliert in "Anforderungen an die Hygiene bei der Aufbereitung von Medizinprodukten" (Bundesgesundheitsblatt 2012-55: 1244-1310, Springer-Verlag 2012) beschrieben.

Dabei wird die Trocknung durch den Sachverhalt erschwert, dass neben Metallen und Glas auch thermolabile Kunststoffe und Gummi vorliegen. Kunststoffe weisen einerseits relativ hohe spezifische Wärmekapazitäten [kJ/kg·K] (Polyethylen: 2,1; Gummi: 1,4-1,7; Teflon/Silikon: 1,0) auf im Vergleich zu Edelstahl (0,5-0,7) und Glas (0,6-0,8). Dennoch behindern Kunststoffe aufgrund deren geringen Wärmeleitfähigkeiten [W/(m·K)] (Polyethylen: 0,3-0,6; Gummi: 0,16; Teflon/Silikon: 0,25) den für die Erwärmung von Restwassertropfen/ -filmen notwendigen Energieübergang und damit eine schnelle Verdunstung bzw. Trocknung erheblich.

Um die Trocknungswirkung von RDG insbesondere bei Kunststoffoberflächen zu verbessern, wurde nach DE 3143005 A1 erstmals eine Drehtrommel integriert. Durch deren Drehbewegung werden auf dem Material verbliebene Wassertropfen/-filme auf andere Kunststoffoberflächen verschoben, dort stärker erwärmt bzw. verdunstet und so eine wirksamere Trocknung erreicht.

Weiterhin zielten DE 102007003894 A1 sowie EP 2656767 A1 darauf ab, die im RDG zur Verteilung der Spülflotte vorhandenen Sprüheinrichtung auch zur Einströmung von Heißluft und damit zur Verbesserung der Trocknungswirkung zu nutzen.

Aus anderen Anwendungen sind Trocknungsverfahren insbesondere für thermolabile Produkte bekannt (DE 10358260 B4), bei welchen zunächst die absolute Luftfeuchte der Zuluft auf < 1 g/kg reduziert und diese dann in das Trockengut eingebracht wird, was ebenfalls eine Beschleunigung der Trocknung erreicht. Dieses Verfahren wäre auch für die Trocknung in RDG nutzbar.

Mit DE 10 2014 008 171 A1 wurde ein gegenüber Druck und Vakuum stabiler RDG beschrieben, in welchem die Trocknung mittels Unterdruck möglich wird. Dabei erfolgt die Desinfektion mit Dampf bei > 100 °C und es schließt als unmittelbar Prozessschritt eine Vakuumtrocknung an. Mit diesem Verfahren ist für Metallinstrumente, auch komplexe, filigrane, englumige oder solche mit engen Gelenkspalten, von einer den normativen Anforderungen entsprechenden Trocknung innerhalb weniger Minuten auszugehen. Dagegen kann auch dieses Verfahren eine vollständige Trocknung der thermolabilen Kunststoffe nicht sicherstellen:
Da nach Anlegen eines Vakuums Teile der Wassertropfen/-filme verdampfen, wird dem verbleibenden Restwasser viel Wärmeenergie entzogen und es kühlt dabei ab. Aufgrund der sehr geringen Wärmeleitung der Kunststoffe erhält das Restwasser nur so sehr verzögert weitere Energie, dass es während der eingeschränkt verfügbaren Trocknungszeit auf der Kunststoffoberfläche verbleibt ohne zu verdampfen.

Die EP 0 301117 A1 offenbart eine Einrichtung zur Gefriertrocknung mit einer evakuierbaren Kammer und einem Evakuierungssystem. Die Einrichtung ist mit einer zusätzlichen Kondensationsfläche ausgerüstet, die über ein Ventil an einen Vorratsbehälter mit einem niedrigsiedenden Kältemittel angeschlossen ist. Fällt infolge eines Störfalles die Pumpleistung des Evakuierungssystems aus, wird das Ventil geöffnet, so dass die zusätzliche Kondensationsfläche die Pumpwirkung übernimmt.

Auf der Grundlage dieses erkannten Defizites im Stand der Technik ist es die Aufgabe der vorliegenden Erfindung, ein Verfahren und eine Vorrichtung zu schaffen, mit denen eine vollständige Trocknung auch von Gegenständen mit geringen Wärmeleitfähigkeiten, wie z.B. thermolabilen Kunststoffen, in einer sehr kurzen Behandlungszeit erreicht wird.

Diese Aufgabe wird durch die in den Patentansprüchen 1 und 3 angegebenen Merkmale gelöst. Vorteilhafte Ausgestaltungen und Weiterbildungen der Erfindung sind den Unteransprüchen zu entnehmen.

Grundidee der Erfindung ist es, an eine gegenüber Vakuum (< 10 mbar) und Überdruck (> 0,01 bar bis >3 bar) stabile und von außen beheizbaren (Doppelmantel-) Kammer, in der sich die zu trocknenden Materialien befinden, eine Vorrichtung anzubringen, die bei allen noch vorhandenen Wassertropfen/-filmen eine Dampfexplosion erzeugt.

Unter einer Dampfexplosion bzw. einem Dampfexplosionsverfahren ist hierbei die sehr schnelle Verminderung des Drucks mit der Folge einer relativen lokalen Überhitzung und nachfolgend eines Siedeverzuges zu verstehen. Durch den so gezielt induzierten Siedeverzug und der dabei stattfindenden Dampfexplosion werden Teile der (primären) Flüssigkeitstropfen einerseits unmittelbar verdampft. Andererseits werden die primären Flüssigkeitstropfen als wesentlich kleinere sekundäre Flüssigkeitstropfen/-filme verteilt und benetzen mit hoher Geschwindigkeit umgebende, wärmere Oberflächen, wie z. B. Kunststoffoberflächen oder gelangen direkt auf die innere Metalloberfläche des beheizten Doppelmantels der Kammer. Dort nehmen diese kleinsten Flüssigkeitstropfen - relativ zu ihrer geringeren Tropfenmasse - erhebliche zusätzliche Energien aus den sekundär benetzten Oberflächen auf. Ebenso erhalten die sekundären Tropfen zusätzliche Energie aus der Strahlungswärme der beheizten Kammerwände des Autoklaven. Diese zusätzlich aufgenommene Energie steht dann für einen wiederholten Dampfexplosionsvorgang zur Verfügung und erreicht jeweils eine weitere Verdunstung bzw. Minimierung der noch verbleibenden Tropfengrößen.

Mehrfach aufeinanderfolgend durchgeführte Dampfexplosionen führen abschließend zu einer, im Vergleich zum vorbeschriebenen Stand der Technik, vollständigen Verdampfung bzw. Trocknung der Oberflächen in wesentlich kürzerer Prozesszeit. Dabei wird durch eine mit ausreichendem Volumen dimensionierte Vakuumkammer, die den Dampf aus der Trockenkammer mit hoher Geschwindigkeit durch die Rohrleitung abführt, sichergestellt, dass Rückströmungen aus der Rohrleitung in die Trockenkammer und damit Rekontaminationen der zu trocknenden Materialien sicher ausgeschlossen wird.

Bei der für das Dampfexplosionsverfahren geeigneten Trocken- (Autoklaven-) Kammer kann es sich z. B. um eine gegenüber Druck bzw. Vakuum stabile RDG-Kammer entsprechend DE 10 2014 008 171 A1 handeln. Die Anwendung des Dampfexplosionsverfahrens ist aber auch in anderen Autoklaven möglich die völlig unabhängig von den Aufgaben z. B. der Reinigung und Desinfektion, ausschließlich der schnellen Materialtrocknung dienen, insbesondere der von Kunststoffen.

Vorzugsweise verfügt die Trockenkammer über einen z.B. mit Dampf beheizten Doppelmantel, so dass Strahlungswärme über die Innenwände auf das zu trocknende Material wirken kann.

Dabei befinden sich die zu trocknenden z. B. thermostabilen Gegenstände, ggf. gemeinsam mit thermolabilen Materialien, auf einer sie tragenden Halterung, z.B. einem Korbwagen oder einem fahrbaren Spülträger in der gesättigten Dampfatmosphäre der Trockenkammer. Die Trockenkammer ist über ein oder mehrere Ventile und eine Rohrleitung mit einer Vakuumkammer verbunden. Durch eine Vakuumpumpe stehen dabei Vakuumkammer und Rohrleitung in relativem Unterdruck zur Trockenkammer.

Werden die Ventile zwischen der Trockenkammer und der Rohrleitung bzw. der Vakuumkammer sehr schnell geöffnet, so strömt der in der Trockenkammer befindliche Wasserdampf entsprechend dem Druckgefälle in Richtung der Vakuumkammer und wird mittels einer Vakuumpumpe entfernt. Erfolgt diese Druckminderung in der Trockenkammer ausreichend schnell, so werden durch den dann eintretenden Siedeverzug die Restwassertropfen zu einem Anteil verdampfen bzw. sich explosionsartig auf andere Oberflächen, wie z. B. der zu trocknender Materialien oder der beheizten Innenwand der Doppelkammer), verteilen.

Vorzugsweise kann die Summe des Volumens aus Rohrleitung und Vakuumkammer kleiner sein als das Volumen der Trockenkammer.

Das für die Dampfexplosion notwendige Gesamtvolumen aus Rohrleitung und Vakuumkammer kann insbesondere dadurch minimiert werden, dass in der Rohrleitung zwischen den beiden Kammern eine oder mehrere Sprühdüsen eingebracht werden, über die mittels Ventilen kaltes Wasser (z.B. vollentsalztes (VE-) Wasser) in die Rohrleitung gesprüht wird. Dabei können die Düsen das (VE-) Wasser in Richtung der Rohrleitung (horizontal) oder von der Außenwand des Rohres zur gegenüberliegenden Außenwand, und damit senkrecht zur Richtung der Rohrleitung (vertikal) einsprühen. Durch die kalten Wasseraerosole in der Rohrleitung kondensiert der in Richtung Vakuumkammer schnell strömende Wasserdampf aus und bewirkt eine Volumenreduktion: Ein Wasserdampfvolumen von ca. 1.700 Litern bei Normaldruck reduziert sich auf 1 Liter Kondensat und erzeugt damit einen zusätzlichen lokalen Unterdruck, der in Folge bewirkt, dass der Wasserdampf aus der Trockenkammer nochmals beschleunigt nachströmt und dadurch den Effekt der Dampfexplosion zusätzlich und wesentlich verstärkt.

Vorzugsweise können im Rohr zwischen der Trockenkammer und der Vakuumkammer mehrere solcher Sprühdüsen hintereinander angeordnet sein, so dass möglichst der gesamte in der Rohrleitung strömende Wasserdampf kondensiert, den Unterdruck verstärkt und es ermöglicht, das Volumen der Vakuumkammer kleiner auszulegen.

Vorzugsweise ist in der Rohrleitung unmittelbar vor jeder Sprühdüse ein Leitblech installiert, das der Gleichrichtung der turbulenten Dampfströmung dient.
In Strömungsrichtung hinter der Sprühdüse befindet sich vorzugsweise eine (Metall-) Wabe, die eine größere Oberfläche erzeugt auf der das eingesprühte (VE-) Wasser in einen intensiveren Kontakt mit dem versprühten Wassers gelangt und somit eine wesentliche Beschleunigung des Kondensationseffektes erreicht.

Vorzugsweise wird die Rohrleitung, die sich zwischen der Trockenkammer und der Vakuumkammer befindet, auf der gesamten Länge oder einer Teillänge als Doppelmantel aufgeführt und es strömt eine Kühlflüssigkeit durch den äußeren Mantel der Rohrleitung. Dabei bewirkt die Kühlung der inneren Rohrleitungs-Oberfläche eine weitere Beschleunigung der Kondensation des im Innenrohr strömenden Dampfes. Auch hierdurch wird die Wirkung der Dampfexplosion verstärkt und das Volumen der Vakuumkammer kann minimiert werden.

Vorzugsweise weist die Trockenkammer vor der Durchführung des Dampfexplosionsverfahrens zunächst einen Druck von ca. 1.000 mbar auf, während der Druck in der Rohrleitung sowie in der Vakuumkammer < 200 mbar, vorzugsweise < 80 mbar bis < 30 mbar beträgt.

Vorzugsweise sprüht bereits (VE-) Wasser, das unter einem Überdruck steht mit einer Temperatur < 15°C (< 95°C) in die Rohrleitung ein und bildet dort einen Sprühnebel, bevor die schnelle Öffnung der Ventile zwischen Trockenkammer und der Rohrleitung zur Vakuumkammer erfolgt. Dabei können sich eine oder mehrere Sprühdüsen hintereinander geschaltet in der Rohrleitung befinden, deren Sprühwinkel bevorzugt 45° (> 10°) beträgt.

Vorzugsweise verfügt die Trockenkammer über mehrere Ventile mit Verbindung zur Rohrleitung bzw. der Vakuumkammer, um zu einem definierten Zeitpunkt der gemeinsamen Öffnung eine sehr kurzfristige Druckänderung zur Erzeugung eines Siedeverzuges auszulösen und so die noch in der Trockenkammer vorhandenen Wassertropfen explosionsartig zu verteilen bzw. verdampfen.

Vorzugsweise wirkt die zur Erreichung einer maximalen Dampfexplosion erforderliche sehr kurzfristige Druckminderung auch über die Kupplungsvorrichtung des Spülgutmaterialträgers (Korbwagen). Dabei wird das Vakuum von Rohrleitung bzw. Vakuumkammer mit Öffnung des Ventils den Dampf in der Trockenkammer (RDG: Spülraum) auch über die Dreharme bzw. drehbaren Sprüheinrichtungen etc. absaugen, entgegen der Strömungsrichtung der Reinigungsflotte. Dies bewirkt, dass für die Dampfexplosion auch die Leitungsquerschnitte der Spülgutmaterialträger (Korbwagen) über die Kupplungsvorrichtungen für eine schnelle Einwirkung des Vakuums genutzt werden. Weiterhin können durch diese Ausgestaltung auch in den Rohren der Spülgutmaterialträger (Korbwagen) verbliebene Wassertropfen einer Dampfexplosion zugeführt, verteilt und schnell als Dampf abgesaugt werden.

Vorzugsweise können an Stelle einer Trockenkammer auch mehrere Trockenkammern über Ventile an eine Einheit aus Rohrleitung und Vakuumkammer angeschlossen werden.

Vorzugsweise weist die Rohrleitung von der Vakuumkammer bis zur Trockenkammer ein Gefälle von z. B. 1/100 bis 5/100 auf, so dass das versprühte Wasser sowie entstehendes Kondensat schnell in Richtung der Dampfströmung abfließen kann.

Vorzugsweise weist die Vakuumkammer Melkventile auf, so dass das dort gesammelte Wasser abgeführt werden kann, ohne dass das Volumen der Vakuumkammer durch sich darin ansammelndes Wasser reduziert wird. Um einen relevanter Druckanstieg in der Vakuumkammer auszuschließen, öffnet zunächst nur das obere Melkventil, das unmittelbar an die Vakuumkammer angrenzt, so dass Wasser mittels Schwerkraft in das anschließende Rohrstück fließen kann. Danach schließt das obere Melkventil und es öffnet das untere Melkventil, so dass das Wasser aus dem Rohrstück frei fließen kann. Durch mehrfache Abfolge bewirken die beiden Melkventile eine Entleerung der Vakuumkammer.

Im Folgenden wird die Erfindung anhand von Ausführungsbeispielen im Zusammenhang mit der Zeichnung ausführlicher erläutert und es werden eigene Untersuchungsergebnisse vorgestellt.

Es zeigen:
- Fig. 1: eine Vorrichtung nach einem ersten Ausführungsbeispiel der Erfindung;
- Fig. 2: eine Vorrichtung nach einem zweiten Ausführungsbeispiel der Erfindung;
- Fig. 3: eine Vorrichtung nach einem dritten Ausführungsbeispiel der Erfindung;
- Fig. 4: eine Vorrichtung nach einem vierten Ausführungsbeispiel der Erfindung; und
- Fig. 5: eine Vorrichtung nach einem fünften Ausführungsbeispiel der Erfindung.

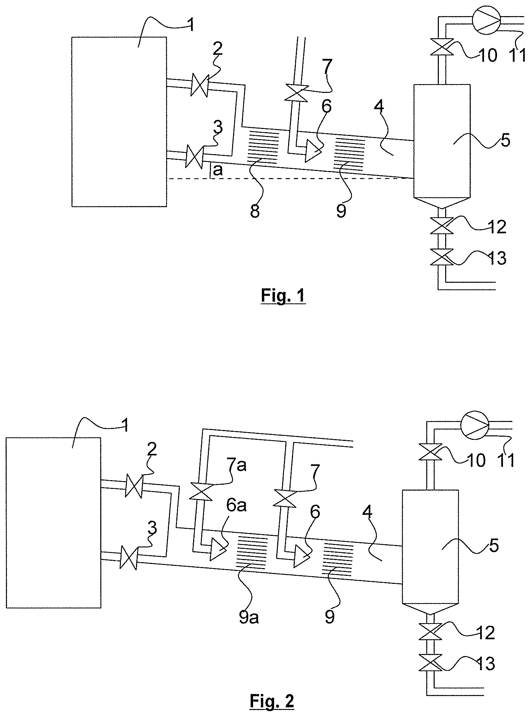

Figur 1 zeigt eine gegenüber Überdruck und Vakuum stabile Trockenkammer 1 mit einem Volumen V-1, das beispielsweise ca. 450 l beträgt und im Betrieb einen Absolutdruck von ca. 1050 mbar aufweist. Die Trockenkammer 1 ist über ein oder mehrere Ventile 2, 3 mit einer Rohrleitung 4 verbunden, die in eine Vakuumkammer 5 mündet. An die Rohrleitung 4 ist eine Einspritzdüse 6 angeschlossen, die (VE-)Wasser über ein Ventil 7 einbringt. Die Einspritzdüse 6 ist vorzugsweise horizontal ausgerichtet, spritzt also im Wesentlichen parallel zur Längsrichtung der Rohrleitung 4.

In Strömungsrichtung vor der Einspritzdüse 6 ist ein Leitblech 8 installiert, das eine Strömungsstabilisierung des aus der Trockenkammer turbulent zuströmenden Dampfes bewirkt. Stromabwärts hinter der Einspritzdüse 6 befindet sich eine (Metall-) Wabe 9, welche eine Oberflächenvergrößerung der Rohrleitung 4 erreicht.

An die Vakuumkammer 5 ist über ein Ventil 10 eine Vakuumpumpe 11 angeschlossen, die einen Unterdruck von ca. 80 mbar erzeugt sowie zwei Melkventile 12 und 13, über die Flüssigkeit abgeführt werden kann. Die Rohrleitung 4 weist vorzugsweise ein Gefälle (Winkel a) von der Trockenkammer 1 zur Vakuumkammer 5 auf.

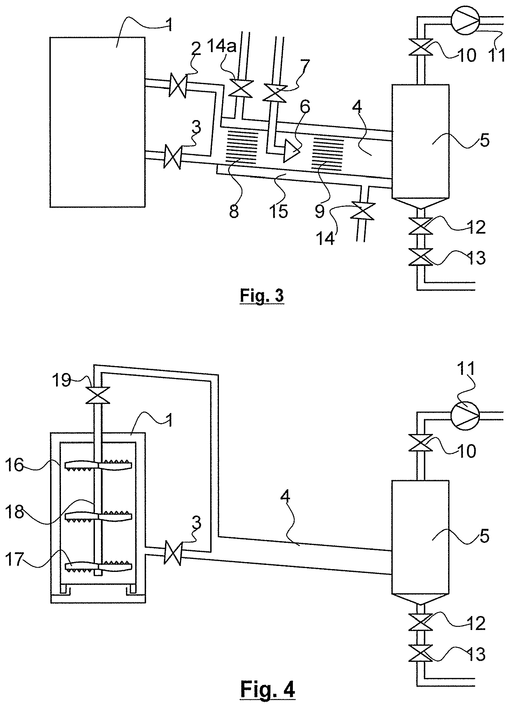

In der Trockenkammer 1 befindet sich auf einem ein- und ausfahrbaren Korbwagen 16 (Fig. 4) zu trocknendes Gut, wie insbesondere thermolabile Kunststoffmaterialien mit einer Temperatur von ca. 100°C in einer Wasserdampfatmosphäre mit einem Druck von ca. 1.050 mbar (ca. Atmosphärendruck) und weist noch eine Restfeuchtigkeit in Form von Wassertropfen und/oder Wasserfilmen auf.

Die Trockenkammer 1 ist über die beiden geschlossenen Ventile 2 und 3 und ein geschlossenes Ventil 19 (Fig. 4) zur Kupplungsvorrichtung des Korbwagens 16 (zu den Düsen, Dreharmen, Sprüheinrichtungen) mit einer Rohrleitung 4 an die Vakuumkammer 5 angeschlossen.

Die Rohrleitung 4 hat beispielsweise ein Volumen von 40 l und ein Gefälle von 200:1. Die Vakuumkammer 5 hat beispielsweise ein Volumen von 240 l. Die Vakuumpumpe 11 erzeugt in der Rohrleitung 4 und der Vakuumkammer 5 einen Absolutdruck von ca. 80 mbar, der gegenüber Atmosphäre somit als "Unterdruck" zu bezeichnen ist.

Die Rohrleitung 4 weist in ihrem runden InnenQuerschnitt (100 mm) in der Querschnittsmitte horizontal wirkende Einspritzdüse 6, 6a auf (Sprühwinkel: 45°; Durchmesser: 2 mm), die (VE-) Wasser (Druck: 3,5 bar) in Richtung der Vakuumkammer sprühen. Weiterhin können sich unter- und oberhalb des Auslasses der ersten horizontalen Einspritzdüse 6 weitere zwei nicht dargestellte vertikale Düsen (Sprühwinkel: 120°; Durchmesser: 2 mm) befinden, die gegenüberliegend auf dem Rand der Rohrleitung 4 positioniert sind und ebenfalls (VE-) Wasser (Druck: 3,5 bar) freisetzen.

Im Abstand von ca. 150 mm in Strömungsrichtung hinter den horizontalen Einspritzdüse 6, 6a befinden sich rechteckigen Waben von ca. 10 mm Kantenlänge (Stärke: 1 mm, Länge: 55 mm), auf die das versprühte Wasser auftrifft, die Waben-Oberflächen benetzt und kühlt (Fig. 1, 2).

Gemäß Fig. 2 können in der Rohrleitung 4 auch mehrere horizontal in die Rohrleitung 4 sprühende Einspritzdüsen 6, 6a mit Waben 9, 9a zur Oberflächenvergrößerung der Rohrleitung 4 vorhanden sein. Die Anschlüsse für (VE-)Wasser zu den Einspritzdüse 6 und 6a sind durch Ventile 7, 7a separat öffen- und schließbar.

Weiterhin kann die Rohrleitung 4 auf einer Länge von 800 mm über einen Doppelmantel 15 verfügen, deren äußerer Mantel den Bereich der Einspritzdüse 6, des Leitblechs 8 sowie der Wabe 9 umgibt. Der äußere Doppelmantel 15 wird von (VE-) Wasser (Temperatur: 15°C) durchströmt (Fig. 3).

Zum Start der ersten Dampfexplosion öffnen zu einem definierten Zeitpunkt alle Ventile 2 und 3 zur Trockenkammer 1 sowie das Ventil 19 zum Korbwagen 16 schnell und gleichzeitig. Dann strömt sofort Dampf aus der Trockenkammer 1 mit hoher Geschwindigkeit turbulent in die Rohrleitung 4 in Richtung Vakuumkammer 5.

Bereits in der Rohrleitung 4 kondensiert der Dampf sowohl an den versprühten (VE-) Wasseraerosolen als auch auf dem gekühlten Doppelmantel 15 und bewirkt dort einen zusätzlichen lokalen Unterdruck (Volumenreduktion Dampf:Kondensat ∼ 1.700:1).

Aufgrund der sehr schnellen Druckminderung verdampft ein Teil der Wassertropfen explosionsartig, bewirkt eine massive Beschleunigung der übrigen Tropfenteilen und verteilt diese als wesentlich kleinere Tropfen auf andere Oberflächen.

Werden die Ventile 2, 3 zur Trockenkammer 1 und das Ventil 19 zur Kupplungsvorrichtung des Korbwagens 16 nach einem Zeitraum von ≤ 5 Sekunden wieder geschlossen, ist damit der erste Dampfexplosionsvorgang beendet. Die Rohrleitung 4 und Vakuumkammer 5 weisen dann einen Absolutdruck von ca. 90 mbar auf und der Druck in der Trockenkammer 1 ist durch die Einwirkung der Dampfexplosion auf ca. 200 mbar abgesunken. Aufgrund der in der Trockenkammer 1 weiterhin stattfindenden Verdampfung der noch vorhandenen Wasserreste der zu trocknenden Materialien steigt der Druck in der Trockenkammer 1 jedoch während der nachfolgenden Ausgleichszeit von ≤ 2 Minuten auf ca. 500-800 mbar an. Dabei ist der nach der Ausgleichszeit erreichte Enddruck von der Gesamtmasse der zu entfernenden Wasserreste abhängig.

Die Durchführung einer zweiten sowie ggf. weiterer Dampfexplosionen erfolgt wie zuvor beschrieben durch schnelles Öffnen der Ventile 2, 3 zur Trockenkammer 1 bzw. des Ventils 19 zum Korbwagen 16, welche die Verbindung zur Rohrleitung 4 bzw. der Vakuumkammer 5 herstellen.

Die notwendige Anzahl aufeinanderfolgender Dampfexplosionen mit schneller Einwirkung des Vakuums und anschließender Ausgleichszeiten erfolgt, bis eine vollständige Materialtrocknung erreicht ist und ist von der Restfeuchte des Materials in der Trockenkammer 1 abhängig.

Gemäß dem Ausführungsbeispiel der Fig. 3 ist die Rohrleitung 4 mit einem Doppelmantel 15 umgeben, dem über ein Ventil 14a Kühlwasser zugeführt und über ein Ventil 14 abgeführt wird. Damit wird die Oberflächentemperatur der Rohrleitung 4 so reduziert, dass der darin strömende Dampf auch darauf schnell kondensiert. Selbstverständlich kann ein solcher Doppelmantel 15 auch bei den anderen Ausführungsbeispielen vorgesehen sein.

Im Ausführungsbeispiel der Fig. 4 hat der in der Trockenkammer 1 befindliche Korbwagen 16 mehrere Dreharme 17, wobei das Vakuum der Vakuumpumpe 11 über eine Rohrleitung 18 und ein Ventil 19 auf die Dreharme 17 wirkt. Damit wird Dampf aus dem gesamten Innenraum der Trockenkammer 1 auch über die Rohrleitung 18 des Korbwagens 16 schnell abgesaugt. Selbstverständlich können auch hier die übrigen Merkmale der Figuren 1 bis 3 zusätzlich vorgesehen sein.

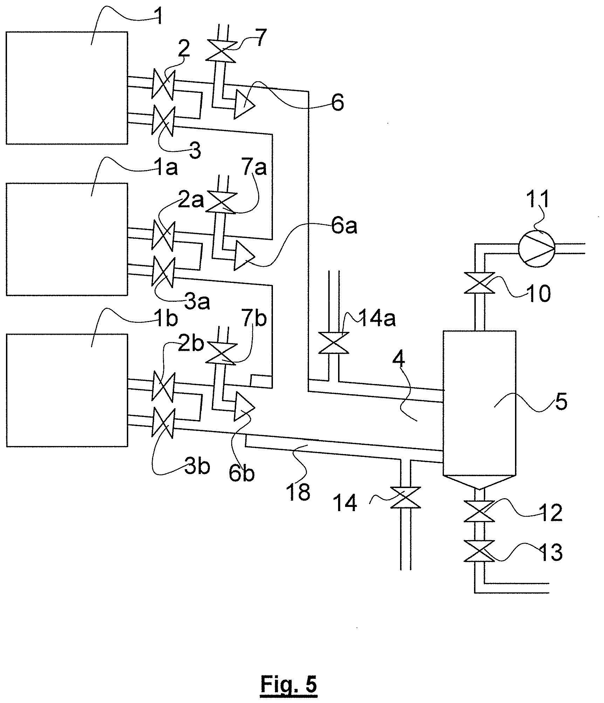

Gemäß dem Ausführungsbeispiel der Fig. 5 können auch mehrere Trockenkammern 1, 1a, 1b an die Rohrleitung 4 angeschlossen sein, wobei jeweils, gemäß Fig. 1, die Trockenkammer 1 über Ventile 2, 3; 2a, 3a, 2b, 3b mit der Rohrleitung 4 verbunden sind, wobei jeweils über die Ventile 7, 7a, 7b (VE-) Wasser durch die Düsen 6, 6a, 6b einsprüht.

Alle übrigen Merkmale entsprechen denen der Ausführungsbeispiele der Figuren 1-4 und können wahlweise implementiert sein.

Zusammenfassend besteht die Vorrichtung aus einer gegenüber Unterdruck (Vakuum) und Überdruck festen Trockenkammer, die über ein oder mehrere schnell wirkende Ventile mit einer Einheit aus Rohrleitung, Vakuumkammer und Vakuumpumpe in Verbindung steht, wobei in der Trockenkammer bei Öffnung der schnell wirkenden Ventile explosionsartig ein Unterdruck erzeugt wird, welcher bei Flüssigkeitsresten auf den zu trocknenden Materialien in der Trockenkammer einen Siedeverzug (Dampfexplosion) auslöst, wobei die Flüssigkeitsreste explosionsartig auf andere, wärmere Oberflächen verteilt werden, von welchen sie Energie aufnehmen und vollständig verdampfen können.

Auf die kurzfristigen Erzeugung des explosionsartigen Unterdrucks folgt eine Ausgleichszeit, in welcher die zu trocknenden Materialien aus ihrer neuen Umgebung Energie aufnehmen können.

Die Einleitung des Siedeverzuges (Dampfexplosion) kann durch schnelles Öffnen aller Ventile zwischen Trockenkammer und Rohrleitung / Vakuumkammer mehrfach aufeinanderfolgend durchgeführt werden, während zwischen den Dampfexplosionen ggf. Ausgleichszeiten geschaltet sind.

Dabei werden die Phasen der kurzfristigen Erzeugung eines explosionsartigen Unterdrucks und ggf. die Phasen der Ausgleichszeit so häufig mehrfach aufeinanderfolgend wiederholt, bis das Material vollständig bzw. den Anforderungen entsprechend getrocknet ist.

Die gegenüber Unterdruck (Vakuum) und Überdruck feste Trockenkammer weist einen beheizbaren Außenmantel, bevorzugt einen Dampf-durchströmten Doppelmantel, auf.

In der Rohrleitung von der Trockenkammer zur Vakuumkammer befinden sich eine oder mehrere Sprühdüsen, die bereits vor der Erzeugung eines "explosionsartigen" Unterdrucks Wasser in die Rohrleitung einsprüht und den danach einströmenden Dampf in der Rohrleitung kondensiert.

Die Sprühdüsen setzen ihr Wasser in Richtung und/oder entgegen der Richtung und/oder senkrecht zur Richtung des in der Rohrleitung strömenden Dampfes frei.

In der Rohrleitung von der Trockenkammer zur Vakuumkammer befinden sich ein oder mehrere Leitbleche vor der Sprühdüse, welche die Richtung des strömenden Dampfs beeinflussen.

In Strömungsrichtung von der Trockenkammer zur Vakuumkammer befindet sich nach der Sprühdüse ein die Oberfläche vergrößernde (Metall-) Wabe, die vom versprühten Wasser benetzt wird und eine wirksamere Kondensation des strömenden Dampfs bewirkt.

Die Rohrleitung von der Trockenkammer zur Vakuumkammer wird ganz oder teilweise von einem äußeren Mantel (Doppelmantel) umgeben, der von Kühlwasser durchströmt wird, so dass in der Rohrleitung strömender Dampf an der Oberfläche der Rohrleitung wirksamer kondensiert.

Die zur Einleitung des Siedeverzuges (Dampfexplosion) erforderliche sehr kurzfristige Druckminderung erfolgt auch über Ventile, welche eine Ableitung des Dampfes über die Kupplungsvorrichtung des Materialträgers (Korbwagen) bzw. dessen Dreharme bzw. drehbaren Sprüheinrichtungen bewirken.

Rohrleitungen von der Trockenkammer sind mit Gefälle zur Vakuumkammer verlegt, so dass Flüssigkeiten (Kondensat) zur Vakuumkammer frei abfließen können.

Es können auch mehrere Trockenkammern über separate Ventile an die Rohrleitung zur Vakuumkammer und Vakuumpumpe angeschlossen sein.

Die Vakuumkammer wird durch übereinander angeordnete Ventile entleert, die nacheinander öffnen und schließen (Melkventil), so dass Flüssigkeit aus der Vakuumkammer aufgrund der Gravitation abfließen kann, ohne dass die Vakuumkammer belüftet wird.

Eine Regelungs- und Steuerungseinheit überwacht den gesamten Prozessablauf zur Erzielung von Siedeverzügen.

## Patentansprüche

1. Vorrichtung zur Trocknung von Gegenständen mit einer gegen Über- und Unterdruck festen Trocknungskammer (1) die über mindestens ein Ventil (2, 3, 19) mit einer Rohrleitung (4) verbunden ist, wobei die Rohrleitung (4) mit einer Vakuumkammer (5) verbunden ist und die Vakuumkammer (5) über ein Ventil (10) mit einer Vakuumpumpe (11) verbunden ist und über mindestens einen Melkventil (12, 13) mit einem Auslass verbunden ist,
wobei die Ventile (2, 3, 19) schnell wirkend sind, um schlagartig einen Unterdruck in der Trockenkammer (1) zu erzeugen und damit einen Siedeverzug auszulösen, wodurch Flüssigkeitsreste auf in der Trockenkammer (1) angeordneten Gegenständen auf andere Oberflächen in der Trockenkammer (1) verteilt werden, von welchen sie Energie aufnehmen und weitestgehend vollständig verdampfen, wobei in der Rohrleitung (4) von der Trockenkammer (1) zur Vakuumkammer (5) eine oder mehrere Einspritzdüsen (6, 6a) angeordnet sind, über die Wasser in die Rohrleitung (4) einsprühbar ist.

2. Vorrichtung nach Anspruch 1, **dadurch gekennzeichnet, dass** eine Steuereinrichtung die Ventile (2, 3, 19) zwischen der Trockenkammer (1) und der Vakuumkammer (5) mehrfach aufeinanderfolgend öffnet und schließt.

2. Vorrichtung nach einem der Ansprüche 1 bis 2, dann gekennzeichnet, dass die Trockenkammer (1) einen beheizbaren Außenmantel aufweist, der bevorzugt ein von Dampf durchströmter Doppelmantel ist.

3. Vorrichtung nach einem der vorstehenden Ansprüche, **dadurch gekennzeichnet, dass** die Einspritzdüse(n) Wasser in Richtung und/oder entgegen der Richtung und/oder senkrecht zur Richtung des in die Rohrleitung (4) strömenden Dampfs einsprühen.

4. Vorrichtung nach einem der Ansprüche 1 bis 4, **dadurch gekennzeichnet,**
**dass** in der Rohrleitung (4) ein oder mehrere Leitbleche (8) angeordnet sind, die in Strömungsrichtung vor der Einspritzdüse (6) angeordnet sind.

5. Vorrichtung nach einem der Ansprüche 1 bis 5, **dadurch gekennzeichnet, dass** in Strömungsrichtung von der Trockenkammer (1) zur Vakuumkammer (5)
stromabwärts der Einspritzdüse (6) eine Wabe (9) angeordnet ist, die von dem versprühten Wasser aus der Einspritzdüse (6) benetzt und gekühlt ist.

6. Vorrichtung nach einem der Ansprüche 1 bis 6, **dadurch gekennzeichnet, dass** die Rohrleitung (4) ganz oder teilweise von einem äußeren Doppelmantel (15) umgeben ist, der von Kühlwasser durchströmt ist.

7. Vorrichtung nach einem der Ansprüche 1 bis 7, **dadurch gekennzeichnet, dass** die Rohrleitung (4) in Richtung von der Trockenkammer (1) zur Vakuumkammer (5) mit einem Gefälle (a) verlegt ist.

8. Vorrichtung nach einem der Ansprüche 1 bis 8, **dadurch gekennzeichnet, dass** mehrere Trockenkammern (1, 1a, 1b) über separate Ventile (2,3, 19; 2a, 3a; 2b, 3b) an die Rohrleitung (4) angeschlossen sind.

9. Vorrichtung nach einem der Ansprüche 1 bis 9, **dadurch gekennzeichnet, dass** die Vakuumkammer (5) über zwei hintereinander angeordnete Melkventile (12, 13), die nacheinander öffen- und schließbar sind, entleerbar ist, so dass Flüssigkeit aus der Vakuumkammer (5) aufgrund Gravitation abfließt.

10. Vorrichtung nach einem der Ansprüche 1 bis 10, **gekennzeichnet durch** eine Regelungs- und Steuereinheit, die alle Ventile ansteuert.

## Claims

1. Apparatus for drying items with a excess and low pressure resisting drying chamber (1) that is connected via at least one valve (2, 3, 19) with a pipeline (4), wherein the pipeline (4) is connected to a vacuum chamber (5) and the vacuum chamber (5) is connected to a vacuum pump (11) via a valve (10) and via a suction valve (12, 13) to an outlet,
wherein the valves (2, 3, 19) are fast-acting to abruptly generate a a low pressure in the drying chamber (1) and therewith initiate a delay in boiling, whereby remaining liquids on items located in the drying chamber (1) will be distributed on other surfaces in the drying chamber (1), from where they adsorb energy and boil almost completely, wherein in the pipeline (4) of the drying chamber (1) to the vacuum chamber (5) one or more injection nozzle (6, 6a) is arranged via which water can be sprayed into the pipeline (4).

2. Apparatus according to claim 1, **characterized in that** a control device successively opens and closes the valves (2, 3, 19) between the drying chamber (1) and the vacuum chamber (2) several times.

3. Apparatus according to one of the claims 1 to 2, **characterized in that** the drying chamber (1) includes a heatable overjacket that is preferably a double jacket.

4. Apparatus according to one of the preceding claims, **characterized in that** the injections nozzle(s) spray water in the direction or against the direction and/or perpendicular to the direction of the steam streaming into the pipeline (4).

5. Apparatus according to one of the claims 1 to 4, **characterized in that** in the pipeline (4) one or more baffles (8) are arranged, that are arranged in streaming direction prior the injection nozzle (6).

6. Apparatus according to one of the claims 1 to 5, **characterized in that** in a comb (9) is arranged in streaming direction from the drying chamber (1) to the vacuum chamber (5) downstream of the injection nozzle (6), that is moistened and cooled by the sprayed water from the out of the injection nozzle (6).

7. Apparatus according to one of the claims 1 to 6, **characterized in that** the pipeline (4) is completely or partly surrounded by an outer double jacket (15) that is streamed by cooling water.

8. Apparatus according to one of the claims 1 to 7, **characterized in that** the pipeline (4) is installed in direction from the drying chamber (1) to the vacuum chamber (5) with a slope (a).

9. Apparatus according to one of the claims 1 to 8, **characterized in that** multiple drying chambers (1, 1a, 1b) are connected via separate valves (2, 3, 19, 2a, 3a, 2b, 3b) to the pipeline (4).

10. Apparatus according to one of the claims 1 to 9, **characterized in that** the vacuum chamber (5) is dischargable via two consecutively arranged suction valves (12, 13) that are successively open- and closable, such that liquid from the vacuum chamber (5) flows off due to gravitation.

11. Apparatus according to one of the claims 1 to 10, **characterized by** an adjustment and control unit that actuates all valves.

## Revendications

1. Dispositif de séchage d'objets, comprenant une chambre de séchage (1) supportant la surpression et la dépression et reliée à une conduite (4) au moyen d'au moins une vanne (2, 3, 19), dans lequel la conduite (4) est reliée à une chambre à vide (5) et la chambre à vide (5) est reliée à une pompe à vide (11) au moyen d'une vanne (10) et est reliée au moyen d'au moins une vanne de décharge (12, 13) à une sortie, dans lequel les vannes (2, 3, 19) agissent rapidement pour générer "brusquement" une dépression dans la chambre de séchage (1) et pour déclencher ainsi une distorsion d'ébullition, les résidus liquides sur des objets disposés dans la chambre de séchage (1) étant répartis sur d'autres surfaces dans la chambre de séchage (1), d'où ils absorbent l'énergie et se vaporisent largement complètement, dans lequel une ou plusieurs buses d'injection (6, 6a) sont disposées dans la conduite (4) allant de la chambre de séchage (1) à la chambre à vide (5), par lesquelles de l'eau peut être pulvérisée dans la conduite (4).

2. Dispositif selon la revendication 1, **caractérisé en ce qu'**un dispositif de commande ouvre et ferme plusieurs fois de suite les vannes (2, 3, 19) entre la chambre de séchage (1) et la chambre à vide (5).

3. Dispositif selon l'une des revendications 1 ou 2, **caractérisé en ce que** la chambre de séchage (1) comprend une enveloppe extérieure chauffable qui est de préférence une double enveloppe traversée par de la vapeur.

4. Dispositif selon l'une quelconque des revendications précédentes, **caractérisé en ce que** la ou les buses d'injection pulvérisent de l'eau dans la direction et/ou dans la direction opposée et/ou perpendiculaire à la direction de la vapeur circulant dans la conduite (4).

5. Dispositif selon l'une quelconque des revendications 1 à 4, **caractérisé en ce qu'**une ou plusieurs tôles de guidage (8) sont disposées dans la conduite (4) en amont de la buse d'injection (6) dans la direction d'écoulement.

6. Dispositif selon l'une quelconque des revendications 1 à 5, **caractérisé en ce qu'**un nid d'abeilles (9) est disposé en aval de la buse d'injection (6) dans la direction d'écoulement depuis la chambre de séchage (1) vers la chambre à vide (5), lequel nid d'abeilles (9) est mouillé et refroidi par l'eau pulvérisée sortant de la buse d'injection (6).

7. Dispositif selon l'une quelconque des revendications 1 à 6, **caractérisé en ce que** la conduite (4) est entièrement ou partiellement entourée par une double enveloppe extérieure (15) traversée par de l'eau de refroidissement.

8. Dispositif selon l'une quelconque des revendications 1 à 7, **caractérisé en ce que** la conduite (4) est posée avec une pente (a) dans la direction allant de la chambre de séchage (1) vers la chambre à vide (5).

9. Dispositif selon l'une quelconque des revendications 1 à 8, **caractérisé en ce qu'**une pluralité de chambres de séchage (1, 1a, 1b) sont reliées à la conduite (4) par des vannes distinctes (2, 3, 19 ; 2a, 3a ; 2b, 3b).

10. Dispositif selon l'une quelconque des revendications 1 à 9, **caractérisé en ce que** la chambre à vide (5) peut être vidée par l'intermédiaire de deux vannes de décharge (12, 13) disposées l'une derrière l'autre, qui peuvent être ouvertes et fermées l'une après l'autre, de sorte que du liquide s'écoule par gravité hors de la chambre à vide (5).

11. Dispositif selon l'une quelconque des revendications 1 à 10, **caractérisé par** une unité de régulation et de commande qui commande toutes les vannes.
